# EUROPÄISCHE PATENTSCHRIFT

(11) **EP 1 465 859 B1**
(45) Veröffentlichungstag und Bekanntmachung des Hinweises auf die Patenterteilung: **03.09.2008**
(21) Anmeldenummer: 02799053.0
(22) Anmeldetag: 06.12.2002
(51) Int. Cl.: C07C 67/03, C07C 69/54

(54) **VERFAHREN ZUR KONTINUIERLICHEN HERSTELLUNG VON ALKYL(METH)ACRYLATEN**
METHOD FOR THE CONTINUOUS PRODUCTION OF ALKYL (METH)ACRYLATES
PROCEDE DE FABRICATION CONTINUE D'ALKYL(METH)ACRYLATES

(30) Priorität: 04.01.2002 DE 10200171
(43) Veröffentlichungstag der Anmeldung: 13.10.2004
(73) Patentinhaber: Evonik Röhm GmbH, 64293 Darmstadt (DE)
(72) Erfinder: ACKERMANN, Jochen, 64295 Darmstadt (DE); GROPP, Udo, 64646 Heppenheim (DE); HILTNER, Horst, 64646 Heppenheim (DE); LAUSCH, Hans-Rolf, Luling, LA 70070 (US); LUNT-RIEG, Ingrid, 60437 Frankfurt (DE); SIEGERT, Hermann, 64342 Seeheim-Jugenheim (DE); CARLOFF, Rüdiger, 64291 Darmstadt (DE)
(86) Internationale Anmeldenummer: PCT/EP2002/013828
(87) Internationale Veröffentlichungsnummer: WO 2003/055837

(56) Entgegenhaltungen:
- EP-A- 0 902 017
- EP-A- 0 968 995
- US-A- 5 072 027

## Beschreibung

### Gebiet der Erfindung

Die vorliegende Erfindung betrifft ein weiteres kontinuierliches Verfahren zur Herstellung von Alkyl(meth)acrylaten (C) durch kontinuierliche Umesterung von Methyl(meth)acrylat (A) mit Alkoholen (B) unter Freisetzung von Methanol (D) gemäß folgender Reaktionsgleichung: 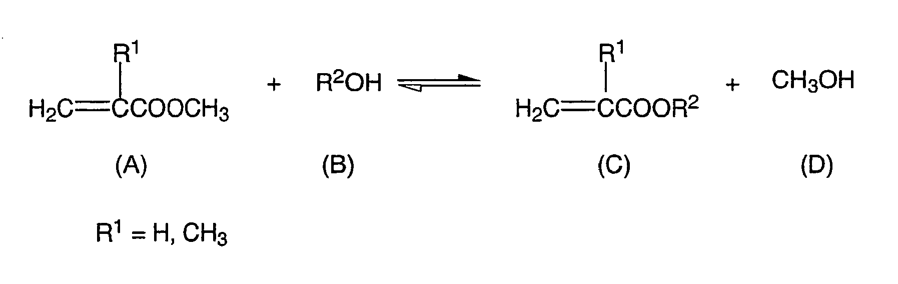 worin R₂ für einen linearen, verzweigten oder cyclischen Alkylrest oder Arylrest mit 2 bis 12 C-Atomen steht. Als Alkohole R₂OH kommen beispielsweise Ethanol, Propanol oder Isopropanol, Butanol oder Isobutanol, Pentanol, Cyclohexanol oder Hexanol, Heptanol, Octanol oder Isooctanol und 2-Ethylhexanol in Frage. Ferner können als Alkohole Isoborneol, Benzylalkohol, Tetrahydrofurfurol, Allylalkohol, Ethylenglycol, 3,3,4-Trimethyl-Cyclohexanol, Phenylethanol, Butylendiglycol, tert.-Butylaminoethanol, Diethylaminoethanol, Ethylentriglycol, Methylentriglycol, Butyldiglycol oder Isopropylidenglycerin verwendet werden.

### Stand der Technik

Alkyl(meth)acrylate können auf verschiedene Verfahrensweisen hergestellt werden:

Durch Umsetzung von Acetoncyanhydrin mit Alkoholen in Gegenwart von Schwefelsäure oder durch Umesterung von Methyl(meth)acrylat mit Alkoholen in Gegenwart eines Katalysators. Bei der Umesterung werden sowohl homogene als auch heterogene Katalysatoren eingesetzt. Häufig wird die Reaktion in Gegenwart von einem Tetraalkyltitanat (Tetraalkoxytitan) durchgeführt. Das bei der Reaktion frei werdende Methanol wird zur positiven Beeinflussung des Reaktionsgleichgewichtes aus dem Reaktor mit Hilfe einer Destillationskolonne aus dem Reaktionsgemisch entfernt.

In der Literatur sind viele diskontinuierlich durchgeführte Umesterungsverfahren (Batch-Umesterungsverfahren) in Verbindung mit unterschiedlichen Katalysatoren beschrieben.

Die Suche nach wirtschaftlicheren Verfahren führte zu der Auffindung von kontinuierlichen Umesterungsverfahren, bei denen die Edukte kontinuierlich zugeführt und die Produkte kontinuierlich abgeführt werden. Die kontinuierlichen Umesterungsverfahren haben gegenüber den diskontinuierlichen Umesterungsverfahren folgende Vorteile: Der Steuer- und Regelaufwand ist geringer, der Personalbedarf ist geringer, die Produktqualität ist besser und weniger schwankend, die Anlagenkapazität erhöht sich aufgrund des Wegfalls des sequenziellen Abarbeitens der einzelnen Herstellungsschritte (Befüllen, Reaktion, Niedrigsiederabtrennung, Produktabtrennung, Entleeren).

Kontinuierliche Umesterungsverfahren sind bekannt.

EP 0 960 877 (Elf Atochem S.A.) beschreibt ein kontinuierliches Verfahren zur Herstellung von Methacrylatestern von Dialkylaminoalkoholen. Man setzt Dialkylaminoalkohole mit im allgemeinen Methyl(meth)acrylat um und erhält das Dialkylaminoalkyl(meth)acrylat nach folgendem Verfahren:

Das Gemisch der Ausgangsstoffe (Methyl(meth)acrylat und Dialkylaminoalkohol) wird zusammen mit einem Tetraalkyltitanat als Umesterungskatalysator (beispielsweise Tetrabutyl-, Tetraethyl- oder Tetra(2-Ethylhexyl)titanat) und mindestens einem Polymerisationsinhibitor (beispielsweise Phenothiazin, tert.-Butylcatechol, Hydrochinonmonomethylether oder Hydrochinon) kontinuierlich einem Rührreaktor zugeführt, wo bei einer Temperatur von 90 - 120°C die Umsetzung zum Dialkylamino(meth) acrylat bei gleichzeitigem kontinuierlichem Abzug des azeotropen Methyl(meth)acrylat/Methanol-Gemisches erfolgt. Das rohe Reaktionsgemisch (Rohester) wird einer ersten Destillationskolonne zugeführt, wobei man bei vermindertem Druck am Kopf der Destillationskolonne einen im wesentlichen katalysatorfreien Strom abzieht und im Sumpf der Destillationskolonne der Katalysator sowie wenig Dialkylaminoalkyl(meth)acrylat abgezogen werden. Der Kopfstrom der ersten Destillationskolonne wird dann einer zweiten Destillationskolonne zugeführt, bei der man unter vermindertem Druck am Kopf einen Strom von niedrigsiedenden Produkten mit wenig Dialkylaminoalkyl(meth)acrylat und im Sumpf einen Strom bestehend aus hauptsächlich Dialkylaminoalkyl(meth)acrylat sowie Polymerisationsinhibitor(en) abzieht, der einer dritten Destillationskolonne zugeführt wird. In der dritten Destillationskolonne wird unter vermindertem Druck eine Rektifikation durchgeführt, in der man am Kopf den gewünschten reinen Dialkylaminoalkyl(meth)acrylatester und im Sumpf im wesentlichen den Polymerisationsinhibitor oder die Polymerisationsinhibitoren abzieht. Der Sumpfstrom der ersten Destillationskolonne wird nach weiterer Aufreinigung mit Hilfe eines Filmverdampfers genauso in den Reaktor zurückgeführt wie der Kopfstrom aus der zweiten Destillationskolonne.

Dieses Verfahren verzichtet auf eine Entwässerung der Alkohole vor dem Einsatz, was zu einer verstärkten Desaktivierung des eingesetzten Tetraalkyltitanats infolge Hydrolyse bis hin zur Bildung unerwünschter Feststoffniederschläge führen kann. Weiterhin verfügt das Verfahren über den Nachteil, dass in der ersten Destillationskolonne der Katalysator im Sumpf bei relativ hohen Temperaturen thermisch beansprucht wird. Dies kann leicht zur Zersetzung des Katalysators führen.
Bei diesem Verfahren werden sowohl die nicht umgesetzten Edukte wie auch das Produkt insgesamt zweimal über Kopf rektifiziert. Dies erfordert sehr hohe Energiekosten und insgesamt 4 Rektifikationskolonnen, die zum Teil sehr groß dimensioniert sein müssen. Der Prozeß ist daher mit sehr hohen Investitions- und Betriebskosten belastet.

EP 0 968 995 (Mitsubishi Gas Chemical Comp.) beschreibt ein kontinuierliches Verfahren zur Herstellung von Alkyl(meth)acrylsäureestem unter Verwendung einer Reaktionskolonne. Die Umesterungsreaktion erfolgt dabei direkt in einer Destillationskolonne (d.h. Reaktor und Destillationskolonne zum Abzug des Methyl(meth)acrylat/Methanol-Azeotrops bilden einen Apparat), der die Ausgangsstoffe (Methyl(meth)acrylat und Alkohol) kontinuierlich zugeführt werden. Der notwendige Katalysator, hier ebenfalls bevorzugt eine Titanverbindung, befindet sich in der Destillationskolonne. Im Fall eines homogenen Katalysators wird der Katalysator in die Destillationskolonne kontinuierlich eindosiert. Die Verwendung von homogenen Katalysatoren in einer Destillationskolonne führt jedoch aufgrund eines Spüleffektes durch den flüssigen Rücklauf in der Destillationskolonne zu einem erhöhten Katalysatorbedarf sowie bei Auftreten eines Katalysatorfeststoffniederschlags zur Verschmutzung der Kolonneneinbauten. Im Fall eines heterogenen Katalysators befindet sich der Katalysator in der Reaktionskolonne. Die Positionierung des Katalysators in der Destillationskolonne ist allerdings nachteilig, weil in der Destillationskolonne dann ein erhöhter Druckverlust auftritt und zusätzlich für die regelmäßige Reinigung der Destillationskolonne ein sehr hoher Aufwand betrieben werden muss. Weiterhin können heterogene Katalysatoren beispielsweise infolge unerwünschter Polymerisation desaktivieren.

### Aufgabe

Aufgabe der vorliegenden Erfindung ist es also, ein kontinuierliches Verfahren zur Umesterung von (Meth)acrylsäuremethylester mit gegenüber Methanol höhersiedenden Alkoholen zur Verfügung zu stellen, das die Nachteile der beiden vorgenannt beschriebenen Verfahren vermeidet. Unter (Meth)acrylsäureestern oder Alkyl(meth)acrylaten werden im folgenden Ester und Derivate der Acrylsäure und der Methacrylsäure verstanden. Weiterhin soll durch das neue Verfahren ein Produkt zur Verfügung gestellt werden, das in der Qualität besser ist als die bisher auf dem Markt befindlichen. Ferner sollen mit dem neuen Verfahren Alkyl(meth)acrylate mit möglichst wenig Aufwand und energetisch günstiger (d.h. kostengünstiger) hergestellt werden.

Gelöst werden diese sowie weitere im einzelnen nicht näher ausgeführte jedoch aus der einleitenden Erörterung des Standes der Technik ohne weiteres erschließbare oder ableitbare Aufgaben durch ein Verfahren mit den Merkmalen des Patentanspruchs 1. Vorteilhafte Abwandlungen des erfindungsgemäßen Verfahrens werden in den auf Anspruch 1 rückbezogenen Ansprüchen unter Schutz gestellt.

### Verfahrensbeschreibung

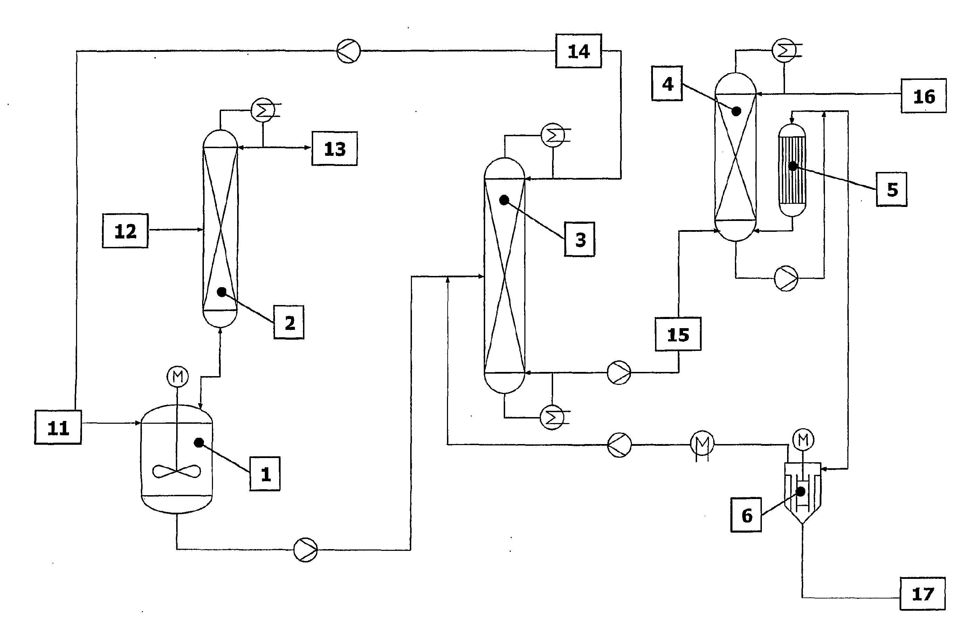

Das Verfahren ist schematisch in Figur 1 dargestellt.

Erläuterungen der Bezugszeichen Figur 1:
1. Reaktionsapparat
2. Azeotrop-Destillationskolonne
3. Niedrigsieder-Destillationskolonne
4. Hochsieder-Destillationskolonne
5. Filmverdampfer
6. Filmverdampfer
11. Methyl(meth)acrylat- und Katalysator-Feed
12. Alkohol-Feed
13. Methanol/Methyl(meth)acrylat-Azeotrop
14. Niedrigsieder-Kreislaufstrom
15. Roh-Ester
16. Rein-Ester
17. Hochsieder und Katalysator

Die Edukte Methyl(meth)acrylat (MMA, 11) und Alkohol (12) werden kontinuierlich einem geeigneten Reaktionsapparat (1) zugeführt, wobei sowohl ein einzelner Reaktionsbehälter als auch eine Kaskade von mehreren hintereinander geschaltenen Reaktionsbehältern eingesetzt werden kann. Es ist sinnvoll, dass alle Reaktionsbehälter einen Brüdenabzug zur Azeotrop-Destillationskolonne (2) zur Entfernung des bei der Reaktion frei werdenden Methanols besitzen.

Das als Katalysator benötigte Tetraalkyltitanat (der Tetraalkoxytitan-Gehalt in Bezug auf eingesetztes MMA beträgt bevorzugt 0,2 - 0,5 Gew.-%) wird wie der/die Polymerisationsinhibitor/en ebenfalls bevorzugt kontinuierlich in den Reaktionsapparat (1) zudosiert. Als Umesterungskatalysatoren können aber auch alle aus dem Stand der Technik bekannten Umesterungskatalysatoren eingesetzt werden. Als Katalysatoren kommen beispielsweise Zirkonacetylacetonat und weitere 1.3-Diketonate des Zirkons in Frage, ferner können Mischungen aus Alkalimetallcyanaten oder Alkalimetallthiocyanaten und Alkalimetallhalogeniden eingesetzt werden, ferner Zinkverbindungen, Erdalkalimetalloxide oder Erdalkalimetallhydroxide, wie beispielsweise CaO, Ca(OH)₂, MgO, Mg(OH)₂ oder Gemische aus den vorstehend genannten Verbindungen, ferner Alkalimetallhydroxide, Alkalimetallalkoxide und Lithiumchlorid und Lithiumhydroxid, es können auch Gemische aus den vorstehend genannten Verbindungen mit den vorstehend genannten Erdalkaliverbindungen und den Li-Salzen eingesetzt werden, Dialkylzinnoxide, Alkalimetallcarbonate, Alkalimetallcarbonate zusammen mit quartären Ammoniumsalzen, wie beispielsweise Tetrabutylammoniumhydroxid oder Hexadecyltrimethylammoniumbromid, ferner Mischkatalysatoren aus Diorganylzinnoxid und Organylzinnhalogenid, saure Ionenaustauscher, Phosphormolybdän-Heteropolysäuren, Titanalkoholate, Chelatverbindungen der Metalle Titan, Zirkonium, Eisen oder Zink mit 1.3-Di-Carbonylverbindungen, Bleiverbindungen, wie beispielsweise Bleioxide, Bleihydroxide, Bleialkoxide, Bleicarbonate oder Bleisalze von Carbonsäuren.

Als Polymerisationsinhibitor kommt beispielsweise Hydrochinonmonomethylether in Verbindung mit Sauerstoff in Frage.

Der eingesetzte Alkohol kann Wasser enthalten. Die Menge an Wasser im eingesetzten Alkohol liegt im Falle von n-Butanol zwischen 50 und 500 ppm (0,05 - 0,005 Gew.-%). Der Alkohol wird vor dem Eintreten in den Reaktionsapparat bevorzugt über die Azeotrop-Kolonne (2) destillativ entwässert. Dabei wird das im Alkohol enthaltene Wasser über Kopf abgezogen. Zur Vermeidung einer Verunreinigung des Methanol/MMA-Azeotrops (13) mit dem eingesetzten Alkohol erfolgt die Aufgabe des Alkohols bevorzugt im unteren Teil der Destillationskolonne (2). Der eingesetzte Alkohol kann auch auf andere Art und Weise entwässert werden:
- durch eine vorgeschaltete Entwässerungs-Destillationskolonne oder
- durch Behandlung mit einem Entwässerungsmittel, wie beispielsweise ein Molekularsieb,
   oder
- durch ein Membrantrennverfahren, wie beispielsweise eine Pervaporation.

Die Entwässerung ist daher bedeutsam, da das im Alkohol enthaltene Wasser zur irreversiblen Schädigung des Katalysators (z.B. Tetraalkyltitanat) im Reaktor führen kann. Durch diesen Entwässerungsschritt vermeidet man die Hydrolyse des Katalysators und die damit entstehenden Kosten durch erhöhte Katalysatoreinsatzmengen und durch Probleme mit Feststoffniederschlägen. Die Umsetzung erfolgt im Reaktionsapparat bei einer Temperatur im Bereich zwischen 80 und 160°C. Bevorzugt ist der Temperaturbereich zwischen 110 und 135°C. Zur positiven Beeinflussung des Reaktionsgleichgewichtes wird das bei der Reaktion frei werdende Methanol über die Destillationskolonne (2) aus dem Reaktionsgemisch als Azeotrop mit MMA abgezogen (13). Die Reaktionsmischung, welche größtenteils aus dem Produkt-Alkyl(meth)acrylat, nicht umgesetztem MMA und Alkohol sowie geringen Mengen Methanol, dem Katalysator, den Polymerisationsinhibitoren und einem sehr geringen Anteil an Nebenprodukten besteht, wird nach ca. 0,5 - 3 Stunden Reaktorverweilzeit (bevorzugt ist eine Verweilzeit von 0,75 - 1,5 Stunden) einer kontinuierlich betriebenen Niedrigsieder-Destillationskolonne (3) zugeführt. Dort erfolgt bei vermindertem Druck, bevorzugt im Bereich von 20 - 200 mbar, die Abtrennung der in Bezug auf den Produktester niedrigsiedenden Komponenten, vorwiegend Methanol, MMA und nicht umgesetzter Edukt-Alkohol. Diese werden über den Kopf der Destillationskolonne abgezogen und in den Reaktorbereich zurückgeführt (14). Durch diesen Kreislaufstrom wird garantiert, dass, bezogen auf den gesamten Prozess, praktisch vollständiger Umsatz in Bezug auf die Edukte MMA und Alkohol vorliegt. Der im Sumpf der Destillationskolonne (3) anfallende mit Katalysator, Polymerisationsinhibitor und hochsiedenden Nebenprodukten noch verunreinigte Roh-Ester (15) enthält vorzugsweise > 98 Gew.-% Produktester und wird zur Aufarbeitung einer weiteren Vakuumdestillationsstufe (4, 5), welche im bevorzugten Druckbereich zwischen 20 und 200 mbar arbeitet, kontinuierlich zugeführt. Hier erfolgt die kontinuierliche destillative Abtrennung des hochreinen Produktesters als Kopfprodukt (16).

Verwendet man zur Abtrennung des Katalysators und der Polymerisationsinhibitoren sowie der hochsiedenden Nebenprodukte aus dem Roh-Ester (15) eine konventionelle Vakuumdestillationskolonne wie im Stand der Technik beschrieben, so kommt es im Sumpf der Kolonne infolge unzulässig hoher thermischer Belastung zu einer Zersetzung des Katalysators und damit zu einer Freisetzung des Eduktalkohols und teilweise auch zu einer Bildung von Ethern des Eduktalkohols.

Beide Verbindungen (Eduktalkohol und Ether des Eduktalkohols) stellen in Bezug auf den Produktester niedrigsiedende Komponenten dar und gelangen somit als Verunreinigung in den Produktester, wodurch die Produktqualität deutlich gesenkt wird. Dieses Problem kann dadurch gelöst werden, dass man zur Abtrennung des Produktesters vom Katalysator und den Polymersiationsinhibitoren sowie den hochsiedenden Nebenprodukten einen Apparat mit schonender Filmverdampfung (5) einsetzt. Als geeignete Apparate sind hierfür Fallfilm-, Dünnschicht- und Kurzwegverdampfer bekannt.

Zum Erreichen der höchsten Produktester-Reinheit (Produktester > 99,9 Gew.-%, Alkohol < 120 ppm, MMA < 10 ppm, Ether < 5 ppm, Farbzahl (Apha) < 1) dient eine weitere, nachgeschaltete Hochsieder-Destillationsstufe (4). Hierbei hat ein einzelner Apparat mit Filmverdampfung aber den Nachteil, eine unzureichende Reinigungsleistung zu bieten, so dass hochsiedende Nebenprodukte mit in den Rein-Produktester (16) gelangen. Dieses Problem wird dadurch gelöst, dass zur Abtrennung der hochsiedenden Nebenprodukte vom Rein-Produktester oberhalb des Apparates mit Filmverdampfung eine Vakuumrektifikationskolonne (4) angeordnet wird.

Nach Abtrennung des Katalysators und der Polymerisationsinhibitoren sowie der hochsiedenden Nebenprodukte aus dem Roh-Ester verbleibt ein gewisser Anteil an Produktester im Sumpfprodukt, damit der Sumpfabstrom noch gut fließ- und förderfähig bleibt. Um bei der Ausschleusung von Katalysator und hochsiedenden Nebenprodukten (17) den Verlust an Produktester zu minimieren, sollte eine Vakuum-Eindampfstufe, welche im bevorzugten Druckbereich von 20 - 200 mbar arbeitet, nachgeschaltet werden (6). Für diese Aufgabe kommt wiederum ein Apparat mit Filmverdampfung in Frage. Als geeignete Apparate sind hierfür wiederum Fallfilm-, Dünnschicht- und Kurzwegverdampfer bekannt. Der an der Eindampfstufe über Kopf abgeführte Produktester erfüllt aufgrund eines unzulässigen Gehaltes an hochsiedenden Komponenten nicht die geforderte Spezifikation für den Rein-Produktester. Weiterhin enthält er aufgrund der thermischen Zersetzung des Katalysators noch Eduktalkohol und teilweise auch Ether des Eduktalkohols. Aus diesen Gründen kann der Destillatstrom mit dem Ziel, den darin enthaltenen Produktester zu gewinnen, nicht direkt in die Hochsieder-Destillationskolonne (4) zurückgeführt werden, vielmehr muss die Rückführung zum Reaktionsapparat (1) oder vorteilhaft zur Niedrigsieder-Destillationskolonne (3) erfolgen, um die Niedrigsieder vor der ersten Eindampfstufe (5) abzutrennen.

Das erfindungsgemäße Verfahren wird durch die nachfolgenden Beispiele näher erläutert, ohne darauf beschränkt zu sein.

Die aufgeführten Beispiele wurden in einer Versuchsanlage im Pilotmaßstab durchgeführt (Stunden-Durchsatz: 6 - 8 kg Feed (MMA und Alkohol) bzw. 5 - 6 kg Produktester). Der Aufbau der Versuchsanlage war wie in Figur 1 dargestellt bzw. in der Verfahrensbeschreibung beschrieben.

Als Reaktionsapparat (1) wurde ein mit Wasserdampf beheizter, nicht mechanisch gerührter Edelstahl-Reaktionskessel mit einem maximalen Füllvolumen von 15 I verwendet. Der Reaktor war über eine Brüdenleitung mit einer oberhalb montierten Azeotrop-Destillationskolonne (2) verbunden. Bei der Azeotrop-Destillationskolonne (Kopfdruck = 1 bar_{abs}) handelte es sich um eine mit H = 2 m Sulzer CY Metalldrahtgewebepackungen bestückte Technikums-Glaskolonne mit einem Durchmesser von D = 0,1 m. In der Kolonnenmitte (H = 1 m) befand sich der Zulauf für den Eduktalkohol. Der Reaktorabstrom wurde einer Niedrigsieder-Destillationskolonne (3) kontinuierlich zugeführt. Bei dieser Destillationskolonne handelte es sich um eine mit H = 3,8 m Sulzer CY Metalldrahtgewebepackungen bestückte Technikums-Vakuumglaskolonne (Kopfdruck = 120 mbar_{abs}) mit einem Durchmesser von D = 0,1 m. Der Zulauf befand sich auf H = 2 m. Die Sumpfbeheizung erfolgte mit Wasserdampf. Der kondensierte Kopfaustrag (Kreislaufstrom) (14) wurde kontinuierlich zum Reaktor zurückgeführt. Statt des in Figur 1 dargestellten Fallfilmverdampfers (5) kam bei der kontinuierlichen Aufarbeitung des Sumpfabstroms der Niedrigsieder-Destillationskolonne (15) ein mit Thermalöl_beheizter Glasdünnschichtverdampfer mit einer Verdampferfläche von A = 0,1 m² zum Einsatz. Die Brüden dieses Glasdünnschichtverdampfers wurden kontinuierlich in eine oberhalb montierte Hochsieder-Destillationskolonne (4) geleitet. Es handelte sich dabei um eine mit H = 0,5 m Sulzer CY Metalldrahtgewebepackungen bestückte Technikums-Vakuumglaskolonne (Kopfdruck = 120 mbar_{abs}) mit einem Durchmesser von D = 0,05 m. Der Sumpfabstrom wurde einem zweiten, kleineren, ebenfalls mit Thermalöl beheizten Glasdünnschichtverdampfer (6) (Kopfdruck = 120 mbar_{abs}) mit einer Verdampferfläche von A = 0,02 m² kontinuierlich zugeführt. Die Brüden dieses zweiten Glasdünnschichtverdampfers wurden auskondensiert und vereinigt mit dem Reaktorabstrom der Niedrigsieder-Destillationskolonne kontinuierlich zugeführt. Der Sumpfabstrom (17) wurde kontinuierlich aus dem Prozess ausgeschleust.
Die Dosierung der Edukte (MMA und Alkohol) erfolgte kontinuierlich mittels Kolben-Dosierpumpen, wobei der Katalysator (Tetraalkyltitanat) im (per Spezifikation wasserfreien) MMA-Feed gelöst zudosiert wurde. Der MMA/Katalysator-Feed wurde dem Reaktor direkt zugeführt, der Alkohol-Feed wurde (auf Kolonneninnentemperatur) vorgewärmt auf die Mitte der Azeotrop-Destillationskolonne aufgegeben.
Die kontinuierliche Zugabe von 50 -100 g/h Stabilisatorlösung (0,2 Gew.-% Hydrochinonmonomethylether in MMA bzw. Produktester) erfolgte mit Hilfe von Schlauchpumpen in den Rücklaufstrom der Destillationskolonnen.
Die kontinuierliche Förderung der Stoffströme zwischen den Anlagenteilen erfolgte entweder mit Hilfe von Kolben-Dosierpumpen oder durch die Saugwirkung des Vakuums. Zwischenbehälter (Puffervolumina) wurden soweit als möglich vermieden.
Die Zusammensetzung der Stoffströme (MMA-, Alkohol-, MeOH- und Produktester-Gehalt) wurde mit Hilfe eines Gaschromatographen bestimmt.

### Beispiel 1 (Kontinuierliche Herstellung von n-Butylmethacrylat):

Zur kontinuierlichen Herstellung von n-Butylmethacrylat (n-BuMA) wurden dem Reaktionskessel 4 kg/h MMA/Katalysator-Feed mit einem Gehalt an Tetra-n-Butyltitanat (Ti(n-OBu)₄) von 0,45 Gew.-% und 2,7 kg/h n-BuOH-Feed zudosiert. Weiterhin floß dem Reaktor der Kreislaufrückstrom vom Kopf der Niedrigsieder-Destillationskolonne kontinuierlich zu (2,8 kg/h mit folgender Zusammensetzung: 1,0 Gew.-% n-BuMA, 38,3 Gew.-% n-BuOH, 57,3 Gew.-% MMA und 3,4 Gew.-% MeOH). Das molare MMA:n-BuOH-Verhältnis im Reaktorzulauf betrug 1,1:1. Bei einer Reaktorverweilzeit von 1 h und einem MMA/MeOH-Azeotropabzug von 1,5 kg/h stellte sich eine Reaktortemperatur von 115 °C ein. Die Zusammensetzung des MMA/MeOH-Azeotrops betrug 82 Gew.-% MeOH, 18 Gew.-% MMA und < 5 ppm n-BuOH. Der resultierende Reaktorabstrom von 8 kg/h setzte sich wie folgt zusammen: 64,6 Gew.-% n-BuMA, 13,5 Gew.-% n-BuOH, 20,3 Gew.-% MMA, 1,3 Gew.-% MeOH und 0,3 Gew.-% Nebenprodukte. Die Raum-Zeit-Ausbeute des Reaktors bezogen auf n-BuMA betrug somit 570 kg/h/m³. Aufgrund nahezu vollständiger Abtrennung der in bezug auf n-BuMA niedrigsiedenden Komponenten wurde im Sumpfaustrag der Niedrigsieder-Destillationskolonne ein Rohester erhalten (5,8 kg/h), der bereits > 99,5 Gew.-% n-BuMA sowie den gesamten Katalysator und Stabilisator enthielt. Die Ausbeute an n-BuOH bezogen auf den gesamten Prozess betrug folglich nahezu 100 %. Die Ausbeute an MMA bezogen auf den gesamten Prozess betrug abzüglich des zuvor einkalkulierten MMA-Verlustes über das MMA/MeOH-Azeotrop ebenfalls nahezu 100 %. Bei einem Eindampfverhältnis (Verhältnis von Brüden- zu Feedstrom) im ersten, größeren Dünnschichtverdampfer von ca. 90 % werden am Kopf der Hochsieder-Destillationskolonne schließlich 5,1 kg/h Rein-n-BuMA mit folgender Zusammensetzung gewonnen: > 99,92 Gew.-% n-BuMA, < 120 ppm n-BuOH, < 10 ppm MMA, < 5 ppm Di-n-Butylether, Farbzahl (Apha) < 0,2. Bei einem Eindampfverhältnis im zweiten, kleineren Dünnschichtverdampfer von ca. 90 % beträgt die Gesamt-Ausschleusung des Prozesses (Katalysator, Stabilisator, hochsiedende Nebenprodukte, n-BuMA) 0,1 kg/h und der Ausbeuteverlust an n-BuMA bezogen auf das produzierte Rein-n-BuMA < 0,5 Gew.-%.

### Beispiel 2 (Kontinuierliche Herstellung von Iso-Butylmethacrylat):

Zur kontinuierlichen Herstellung von Iso-Butylmethacrylat (i-BuMA) wurden dem Reaktionskessel 3,4 kg/h MMA/Katalysator-Feed mit einem Gehalt an Tetra-i-Butyltitanat (Ti(i-OBu)₄) von 0,56 Gew.-% und 2,36 kg/h i-BuOH-Feed zudosiert. Weiterhin floß dem Reaktor der Kreislaufrückstrom vom Kopf der Niedrigsieder-Destillationskolonne kontinuierlich zu (2,4 kg/h mit folgender Zusammensetzung: 6,2 Gew.-% i-BuMA, 35,3 Gew.-% i-BuOH, 56,3 Gew.-% MMA und 2,2 Gew.-% MeOH). Das molare MMA:i-BuOH-Verhältnis im Reaktorzulauf betrug 1,1:1. Bei einer Reaktorverweilzeit von 1,2 h und einem MMA/MeOH-Azeotropabzug von 1,26 kg/h stellte sich eine Reaktortemperatur von 115 °C ein. Die Zusammensetzung des MMA/MeOH-Azeotrops betrug 82 Gew.-% MeOH, 18 Gew.-% MMA und < 5 ppm i-BuOH. Der resultierende Reaktorabstrom von 6,9 kg/h setzte sich wie folgt zusammen: 67,3 Gew.-% i-BuMA, 12,0 Gew.-% i-BuOH, 19,4 Gew.-% MMA, 0,8 Gew.-% MeOH und 0,5 Gew.-% Nebenprodukte. Die Raum-Zeit-Ausbeute des Reaktors bezogen auf i-BuMA betrug somit 516 kg/h/m³. Aufgrund nahezu vollständiger Abtrennung der in bezug auf i-BuMA niedrigsiedenden Komponenten wurde im Sumpfaustrag der Niedrigsieder-Destillationskolonne ein Rohester erhalten (5,0 kg/h), der bereits > 99,5 Gew.-% i-BuMA sowie den gesamten Katalysator und Stabilisator enthielt. Die Ausbeute an i-BuOH bezogen auf den gesamten Prozess betrug folglich nahezu 100 %. Die Ausbeute an MMA bezogen auf den gesamten Prozess betrug abzüglich des zuvor einkalkulierten MMA-Verlustes über das MMA/MeOH-Azeotrop ebenfalls nahezu 100 %. Bei einem Eindampfverhältnis (Verhältnis von Brüden- zu Feedstrom) im ersten, größeren Dünnschichtverdampfer von ca. 90 % werden am Kopf der Hochsieder-Destillationskolonne schließlich 4,5 kg/h Rein-i-BuMA mit folgender Zusammensetzung gewonnen: > 99,9 Gew.-% i-BuMA, < 150 ppm i-BuOH, < 10 ppm MMA, 0 ppm Di-i-Butylether, Farbzahl (Apha) < 0,2. Bei einem Eindampfverhältnis im zweiten, kleineren Dünnschichtverdampfer von ca. 90 % beträgt die Gesamt-Ausschleusung des Prozesses (Katalysator, Stabilisator, hochsiedende Nebenprodukte, i-BuMA) 0,05 kg/h und der Ausbeuteverlust an i-BuMA bezogen auf das produzierte Rein-i-BuMA < 0,5 Gew.-%.

## Patentansprüche

1. Verfahren zur kontinuierlich betriebenen Herstellung von (Meth)acrylsäurealkylestern der Formel (C), 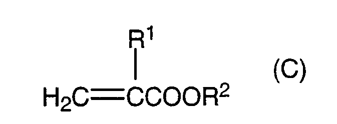 worin R₁ für eine H- oder CH₃-Gruppe und R₂ für einen linearen, verzweigten oder cyclischen Alkylrest oder Arylrest mit 2 bis 12 C-Atomen steht, durch Umsetzung einer Verbindung der Formel (B),
R²OH (B)
wobei R₂ die oben angegebene Bedeutung besitzt, mit Methyl(meth)acrylat (A), 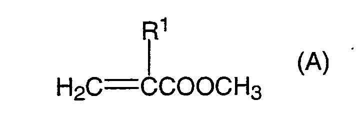 worin R₁ für eine H- oder CH₃-Gruppe steht, in Gegenwart eines Umesterungskatalysators und in Gegenwart mindestens eines Polymerisationsinhibitors in einer Apparatur zur kontinuierlichen Umesterung,
**dadurch gekennzeichnet,**
**dass** die Reaktanden einem geeigneten Reaktionsapparat (1) kontinuierlich zugeführt werden und dass das bei der Reaktion entstehende Methanol als azeotropes Methanol/Methyl(meth)acrylat-Gemisch (13) kontinuierlich mit Hilfe einer Destillationskolonne (2) abgezogen wird und außerdem:
- das Reaktionsgemisch kontinuierlich aus dem Reaktionsapparat in eine Destillationskolonne (3) geführt wird, wobei durch Destillation unter vermindertem Druck über Kopf die leicht flüchtigen Komponenten (A, B, Methanol) und ein sehr geringer Anteil Produktester (C) abgezogen und in den Reaktionsapparat zurückgeführt werden und aus dem Sumpf der Kolonne der Produktester (C) zusammen mit dem Katalysator und den Polymerisationsinhibitoren sowie hochsiedenden Nebenprodukten abgezogen werden;
- der Sumpfstrom (15) aus der Destillationskolonne (3) einem Verdampfer (5) kontinuierlich zugeführt wird, wobei durch Destillation unter vermindertem Druck der Produktester (C) vom Katalysator und den Polymerisationsinhibitoren sowie hochsiedenden Nebenprodukten getrennt wird.

2. Verfahren nach Anspruch 1,
**dadurch gekennzeichnet,**
**dass** der Brüdenstrom des Verdampfers (5) einer Destillationskolonne (4) kontinuierlich zugeführt wird, wobei durch Destillation unter vermindertem Druck über Kopf der hochreine Produktester (C) (16) abgeführt wird und über den Sumpf der Katalysator und die Polymerisationsinhibitoren sowie die hochsiedenden Nebenprodukte mit einem kleinen Teil Produktester (C) abgezogen werden.

3. Verfahren nach Anspruch 1,
**dadurch gekennzeichnet,**
**dass** der Sumpfstrom der Destillationskolonne (4) bzw. des Verdampfers (5) einem weiteren Filmverdampfer (6) kontinuierlich zugeführt wird, wobei durch Destillation unter vermindertem Druck über den Sumpf der Katalysator und die Polymerisationsinhibitoren sowie die hochsiedenden Nebenprodukte abgezogen werden und über Kopf der verbleibende Produktester (C) abgeführt und zur Destillationskolonne (3) oder zum Reaktionsapparat (1) zurückgeführt wird.

4. Verfahren nach Anspruch 1,
**dadurch gekennzeichnet,**
**dass** der Alkohol (B) zur Entwässerung über die Destillationskolonne (2) dem Reaktionsapparat zugeführt wird.

5. Verfahren nach Anspruch 1,
**dadurch gekennzeichnet,**
**dass** das molare Verhältnis von Methyl(meth)acrylat zu Alkohol im Zulauf zum Reaktor zwischen 1 und 2, vorzugsweise 1,05 - 1,15 beträgt.

6. Verfahren nach Anspruch 1,
**dadurch gekennzeichnet,**
**dass** man als Umesterungskatalysator ein Tetryaalkyltitanat einsetzt.

7. Verfahren nach Anspruch 1,
**dadurch gekennzeichnet,**
**dass** der Katalysator in einer Menge von 0,1 - 2 Gew.-%, bezogen auf eingesetztes MMA, eingesetzt wird.

8. Verfahren nach Anspruch 7,
**dadurch gekennzeichnet,**
**dass** der Katalysator in einer Menge von 0,2 -1 Gew.-%, bezogen auf eingesetztes MMA, eingesetzt wird.

9. Verfahren nach Anspruch 1,
**dadurch gekennzeichnet,**
**dass** man als Polymerisationsinhibitor entweder Phenothiazin, Tertiärbutylcatechol, Hydrochinonmonomethylether, Hydrochinon oder deren Gemische einsetzt, wobei die Menge des Inhibitor zwischen 100 und 5000 ppm, bezogen auf das Reaktionsgemisch, beträgt.

10. Verfahren nach Anspruch 1,
**dadurch gekennzeichnet,**
**dass** man als Polymerisationsinhibitor zusätzlich noch Sauerstoff verwendet.

11. Verfahren nach Anspruch 1,
**dadurch gekennzeichnet,**
**dass** man als Alkohol vorzugsweise n-Butanol oder Iso-Butanol verwendet.

12. Verfahren nach Anspruch 1,
**dadurch gekennzeichnet,**
**dass** der Druck in der ersten Destillationskolonne (3) zwischen 20 und 200 mbar beträgt.

13. Verfahren nach Anspruch 1,
**dadurch gekennzeichnet,**
**dass** der Druck in der zweiten Destillationskolonne (4) und in den Filmverdampfern (5) (6) zwischen 20 und 200 mbar beträgt.

14. Verfahren nach Anspruch 1,
**dadurch gekennzeichnet,**
**dass** die Verweilzeit im Reaktionsapparat zwischen 0,5 und 1,5 Stunden beträgt.

15. Verfahren nach Anspruch 1,
**dadurch gekennzeichnet,**
**dass** der Verdampfer (5) ein Filmverdampfer ist.

16. Verfahren nach Anspruch 3,
**dadurch gekennzeichnet,**
**dass** die Verdampfer (5) und der Verdampfer (6) Filmverdampfer sind.

17. Verfahren nach Anspruch 1,
**dadurch gekennzeichnet,**
**dass** man das man als Alkohol 2-Ethylhexanol verwendet.

## Claims

1. Process for continuously preparing alkyl (meth)acrylates of the formula (C) 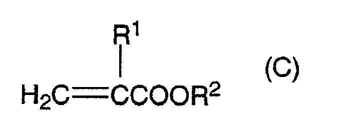 where R₁ is an H or CH₃ group and R₂ is a linear, branched or cyclic alkyl radical or aryl radical having from 2 to 12 carbon atoms by reacting a compound of the formula (B)
R²OH (B)
where R₂ is as defined above with methyl (meth)acrylate (A) 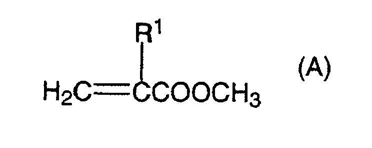 where R₁ is an H or CH₃ group in the presence of a transesterification catalyst and in the presence of at least one polymerization inhibitor in an apparatus for continuous transesterification,
**characterized in that**
the reactants are continuously fed to a suitable reaction apparatus (1) and that the methanol resulting from the reaction is continuously removed as an azeotropic methanol/methyl (meth)acrylate mixture (13) with the aid of a distillation column (2), and also:
- the reaction mixture is continuously conducted from the reaction apparatus into a distillation column (3) in which distillation under reduced pressure is used to remove the volatile components (A, B, methanol) and a very low proportion of product ester (C) overhead which are recycled into the reaction apparatus and to remove the product ester (C) together with the catalyst and the polymerization inhibitors and also high-boiling by-products from the bottom of the column;
- the bottom stream (15) from the distillation column (3) is fed continuously to an evaporator (5) in which distillation under reduced pressure is used to separate the product ester (C) from the catalyst and the polymerization inhibitors and also high-boiling by-products.

2. Process according to Claim 1,
**characterized in that**
the vapour stream of the evaporator (5) is fed continuously to a distillation column (4) in which distillation under reduced pressure is used to remove the highly pure product ester (C) (16) overhead and to remove the catalyst and the polymerization inhibitors and also the high-boiling by-products with a small portion of product ester (C) via the bottom.

3. Process according to Claim 1,
**characterized in that**
the bottom stream of the distillation column (4) and of the evaporator (5) is fed continuously to a further film evaporator (6) in which distillation under reduced pressure is used to remove the catalyst and the polymerization inhibitors and also the high-boiling by-products via the bottom and to remove the remaining product ester (C) overhead which is then recycled to the distillation column (3) or to the reaction apparatus (1).

4. Process according to Claim 1,
**characterized in that**
the alcohol (B) is fed to the reaction apparatus via the distillation column (2) for dewatering.

5. Process according to Claim 1,
**characterized in that**
the molar ratio of methyl (meth)acrylate to alcohol in the feed to the reactor is from 1 to 2, preferably 1.05 - 1.15.

6. Process according to Claim 1,
**characterized in that**
the transesterification catalyst used is a tetraalkyl titanate.

7. Process according to Claim 1,
**characterized in that**
the catalyst is used in an amount of 0.1-2% by weight, based on MMA used.

8. Process according to Claim 7,
**characterized in that**
the catalyst is used in an amount of 0.2-1% by weight, based on MMA used.

9. Process according to Claim 1,
**characterized in that**
the polymerization inhibitor used is either phenothiazine, tert-butylcatechol, hydroquinone monomethyl ether, hydroquinone or a mixture thereof in an amount of 100 to 5000 ppm, based on the reaction mixture.

10. Process according to Claim 1,
**characterized in that**
oxygen is additionally used as a polymerization inhibitor.

11. Process according to Claim 1,
**characterized in that**
the alcohol used is preferably n-butanol or isobutanol.

12. Process according to Claim 1,
**characterized in that**
the pressure in the first distillation column (3) is 20 to 200 mbar.

13. Process according to Claim 1,
**characterized in that**
the pressure in the second distillation column (4) and in the film evaporators (5) (6) is 20 to 200 mbar.

14. Process according to Claim 1,
**characterized in that**
the residence time in the reaction apparatus is 0.5 to 1.5 hours.

15. Process according to Claim 1,
**characterized in that**
the evaporator (5) is a film evaporator.

16. Process according to Claim 3,
**characterized in that**
the evaporator (5) and the evaporator (6) are film evaporators.

17. Process according to Claim 1,
**characterized in that**
the alcohol used is 2-ethylhexanol.

## Revendications

1. Procédé pour la fabrication en continu d'esters alkyliques d'acide (méth)acrylique de formule (C) : 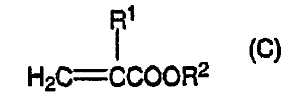 dans laquelle R₁ représente un groupement H ou CH₃ et R₂ représente un radical alkyle ou aryle linéaire, ramifié ou cyclique, ayant 2 à 12 atomes de carbone, consistant à faire réagir un composé de formule (B) :
R²OH (B)
dans laquelle R₂ a la signification indiquée ci-dessus, avec du (méth)acrylate de méthyle de formule (A) : 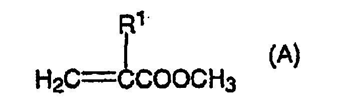 dans laquelle R₁ représente un groupement H ou CH₃, en présence d'un catalyseur de transestérification et en présence d'au moins un inhibiteur de polymérisation dans un appareil permettant d'effectuer la transestérification en continu,
**caractérisé en ce que** :
les réactifs sont acheminés en continu à un appareil réactionnel approprié (1) et **en ce que** le méthanol formé lors de la réaction est extrait sous la forme d'un mélange azéotrope du type méthanol/(méth)acrylate de méthyle (13), de manière continue, à l'aide d'une colonne de distillation (2) et se **caractérise par** ailleurs en ce que :
- le mélange réactionnel est acheminé de manière continue de l'appareil réactionnel vers une colonne de distillation (3), opération pendant laquelle, en réalisant une distillation sous pression réduite, les composants aisément volatils (A, B, méthanol) et une très faible fraction de l'ester produit (C) sont extraits en tête de colonne et recyclés dans l'appareil réactionnel, et l'ester produit (C), conjointement avec le catalyseur et les inhibiteurs de polymérisation, ainsi que les produits secondaires à point d'ébullition élevé, sont extraits en bas de colonne ;
- le courant de bas de colonne (15) provenant de la colonne de distillation (3) est acheminé en continu à un évaporateur (5), opération pendant laquelle l'ester produit (C) est séparé du catalyseur et des inhibiteurs de polymérisation, ainsi que des produits secondaires à point d'ébullition élevé, par distillation sous pression réduite.

2. Procédé selon la revendication 1,
**caractérisé en ce que** :
le courant de fumées de l'évaporateur (5) est acheminé en continu à une colonne de distillation (4), opération pendant laquelle l'ester produit (C) (16) de pureté élevée est évacué par distillation sous pression réduite en tête de colonne, tandis que le catalyseur et les inhibiteurs de polymérisation, ainsi que les produits secondaires à point d'ébullition élevé sont extraits, avec une petite partie de l'ester produit (C), en bas de colonne.

3. Procédé selon la revendication 1,
**caractérisé en ce que** :
le courant de bas de colonne issu de la colonne de distillation (4) ou de l'évaporateur (5) est acheminé en continu à un autre évaporateur à film (6), opération pendant laquelle on effectue l'extraction, par distillation sous pression réduite, du catalyseur et des inhibiteurs de polymérisation, ainsi que des produits secondaires à point d'ébullition élevé, en bas de l'évaporateur, et on évacue l'ester produit restant (C) en tête de l'évaporateur et on le recycle à la colonne de distillation (3) ou à l'appareil réactionnel (1).

4. Procédé selon la revendication 1,
**caractérisé en ce que** :
l'alcool (B) est acheminé à l'appareil réactionnel pour l'élimination de l'eau via la colonne de distillation (2).

5. Procédé selon la revendication 1,
**caractérisé en ce que** :
le rapport molaire du (méth)acrylate de méthyle à l'alcool dans l'alimentation du réacteur est compris entre 1 et 2, de préférence entre 1,05 et 1,15.

6. Procédé selon la revendication 1,
**caractérisé en ce que** :
on utilise un titanate de tétraalkyle comme catalyseur de transestérification.

7. Procédé selon la revendication 1,
**caractérisé en ce que** :
le catalyseur est utilisé en quantité de 0,1 à 2 % en poids par rapport au MMA utilisé.

8. Procédé selon la revendication 7,
**caractérisé en ce que** :
le catalyseur est utilisé en quantité de 0,2 à 1 % en poids par rapport au MMA utilisé.

9. Procédé selon la revendication 1,
**caractérisé en ce que** :
on utilise comme inhibiteur de polymérisation, la phénothiazine, le butylcatéchol tertiaire, le monométhyléther d'hydroquinone, l'hydroquinone ou leurs mélanges, la quantité de l'inhibiteur étant comprise entre 100 et 5000 ppm par rapport au mélange réactionnel.

10. Procédé selon la revendication 1,
**caractérisé en ce que** :
on utilise encore de l'oxygène supplémentaire comme inhibiteur de polymérisation.

11. Procédé selon la revendication 1,
**caractérisé en ce que** :
on utilise, de préférence, le n-butanol ou l'isobutanol, comme alcool.

12. Procédé selon la revendication 1,
**caractérisé en ce que** :
la pression dans la première colonne de distillation (3) est comprise entre 20 et 200 mbars.

13. Procédé selon la revendication 1,
**caractérisé en ce que** :
la pression dans la seconde colonne de distillation (4) et dans les évaporateurs à film (5) et (6) est comprise entre 20 et 200 mbars.

14. Procédé selon la revendication 1,
**caractérisé en ce que** :
le temps de séjour dans l'appareil réactionnel est compris entre 0,5 et 1,5 heure.

15. Procédé selon la revendication 1,
**caractérisé en ce que** :
l'évaporateur (5) est un évaporateur à film.

16. Procédé selon la revendication 3,
**caractérisé en ce que** :
l'évaporateur (5) et l'évaporateur (6) sont des
évaporateurs à film.

17. Procédé selon la revendication 1,
**caractérisé en ce que** :
on utilise du 2-éthylhexanol comme alcool.
